# EUROPEAN PATENT APPLICATION

(11) **EP 4 631 494 A2**
(43) Date of publication of application: **15.10.2025**
(21) Application number: 25185465.9
(22) Date of filing: 27.10.2021
(51) Int. Cl.: A61K 8/92

(54) **COMPOSITIONS SUITABLE FOR USE IN SKIN CARE**

(62) Divisional of application: 21306507.1
(71) Applicant: Kenvue Brands LLC, Summit, NJ 07901 (US)
(72) Inventor: LEFEBVRE, Marjorie, 27100 VAL DE REUIL (FR); GESLIN, Marie Cécile, 27100 VAL DE REUIL (FR); POTIER, Julie, 27100 VAL DE REUIL (FR)
(74) Representative: Carpmaels & Ransford LLP

(57) **Abstract**

Provided are compositions suitable for skin care. Certain compositions comprise
a. one or more a C20-24 fatty alcohols;
b. one or more anionic surfactants;
c. one or more thickening polysaccharides comprising succinoglycan;
d. one or more emollients comprising at least one natural butter having a natural butter which is solid at 20°C and has a melting point below 50°C; and
e. about 50 to about 95 wt.% of water by weight of the total composition, wherein the composition is in the form of an emulsion.

## Description

### FIELD

The invention generally relates to composition for skin care. In particular the invention relates to an emulsion comprising fatty alcohol, anionic surfactant, thickening polysaccharides, emollient and water. The invention also relates to a method of treating the skin using said composition.

### BACKGROUND

Consumers desire skin care compositions for a variety of purposes, including, for example, to provide moisturization and/or cleansing of the skin. Skin care compositions must be capable of being applied to the skin of the user, and often require the presence of an aqueous component, such as water, to allow the user to apply the product to the skin easily and effectively; and an oily phase providing the moisturizing benefit.

However certain skin care compositions may leave a white film of thin foam on the consumer skin providing an unpleasant and unaesthetic soapy effect. Avoiding this effect is challenging and requires a reformulation the oily phase of the emulsion without modifying the rheological properties or the stability of the composition.

In the recent years, consumers demand has evolved toward more natural products. These changes may be driven, for example, by ecological concerns regarding the sourcing of raw material, or by health concerns regarding potential side effects of ingredients such as preservatives.

Manufacturers are constantly trying to improve the formulation of their products to match consumer expectations. Consequently, it is highly desirable to develop moisturizing composition with more sustainable raw material.

There is a need to identify new way to formulate skin care emulsions with sustainable ingredients without this soapy effect while still providing the expected rheology and stability.

### SUMMARY OF THE INVENTION

One aspect of the invention pertains to a composition comprising :
a. one or more a C20-24 fatty alcohols;
b. one or more anionic surfactants;
c. one or more thickening polysaccharides comprising succinoglycan;
d. one or more emollients comprising at least one natural butter having a natural butter which is solid at 20°C and has a melting point below 50°C; and
e. about 50 to about 95 wt.% of water by weight of the total composition,
wherein the composition is in the form of an emulsion.

This aspect may be combined with a variety of embodiments, in any combination. Thus, in some embodiments, the one or more C₂₀₋₂₄ fatty alcohols is selected from the group consisting of behenyl alcohol, arachinyl alcohol, heneicosanol, lignoceryl alcohol and combinations thereof. In one or more embodiments, the one or more C₂₀₋₂₄ fatty alcohols is present in an amount of from about 1 to about 5 wt.% of by weight of the total composition. In some embodiments, the one or more anionic surfactants is selected from the group consisting of potassium cetyl phosphate, glyceryl stearate citrate, fatty acids in the anionic form, and combinations thereof. In one or more embodiments, the one or more one or more anionic surfactants is present in an amount of from about 0.1 to about 3 wt.% of by weight of the total composition. In some embodiments, the one or more thickening polysaccharides further comprises xanthan gum, hydroxyalkyl cellulose, alkyl cellulose, hydroxyalkyl alkyl cellulose, guar gum, carrageenan, alignates, pectin, and combinations thereof. In one or more embodiments, the one or more thickening polysaccharide is present in an amount of from about 0.05 to about 2 wt.% of by weight of the total composition. In some embodiments, at least one natural butter having a natural butter which is solid at 20°C and has a melting point below 50°C is present in an amount ranging from about 1.5 to about 5 wt.%. In one or more embodiments, the at least one natural butter having a natural butter which is solid at 20°C and has a melting point below 50°C comprises shea butter. In some embodiments, the emollient is selected from the group consisting of shea butter, cocoglyceride, candelilla cera and hydrogenated vegetable oil, olus oil; caprylyl glyceryl ether, and combinations thereof. In one or more embodiments, the one or more C₂₀₋₂₄ fatty alcohols comprise behenyl alcohol, the one or more emollients comprises shea butter, and the behenyl alcohol and shea butter are present in a ratio of about 0.5 to about 2. In some embodiments, the composition further comprises a non-ionic surfactant.

Another aspect of the invention pertains to a composition comprising
a. about 1 to about 5 wt.% of one or more C20-24 fatty alcohols by weight of the total composition;
b. about 0.2 to about 3 wt.% of one or more anionic surfactants by weight of the total composition;
c. about 0.05 to about 2 wt.% of one or more thickening polysaccharides comprising succinoglycan by weight of the total composition;
d. about 1 to about 20 wt. % of one or more emollients comprising at least one natural butter having a natural butter which is solid at 20°C and has a melting point below 50°C by weight of the total composition; and
e. about 50 to about 95 wt.% of water by weight of the total composition,
wherein the composition is in the form of an emulsion.

In one or more embodiments, the composition comprises:
a. about 1 to about 5 wt.% of behenyl alcohol by weight of the total composition;
b. about 0.2 to about 3 wt.% of potassium cetyl phosphate by weight of the total composition;
c. about 0.05 to about 1 wt.% of succinoglycan by weight of the total composition;
d. about 0.05 to about 1 wt.% of xanthan gum by weight of the total composition
e. about 1 to about 20 wt. % of one or more emollients comprising shea butter; and
about 70 to about 80 wt.% of water by weight of the total composition.

Another aspect of the invention pertains to a method of treating skin, the method comprising: applying the composition of any of the embodiments described above.

These and other features and advantages of the present invention will be readily apparent from the following detailed description of the invention.

### DETAILED DESCRIPTION OF THE INVENTION

As used herein, a skin care product or skin care composition is a product that can be applied to the skin or hair of a user, providing a benefit to that user. The benefit can be, for example, hydration of the skin or hair or delivery of one or more therapeutic or benefit agents to the skin or hair, as described in more detail below. The skin care product can be, for example, a moisturizer or other skin beneficial product.

One aspect of the invention pertains to a composition comprising:
a. one or more a C20-24 fatty alcohols;
b. one or more anionic surfactants;
c. one or more thickening polysaccharides comprising succinoglycan;
d. one or more emollients comprising at least one natural butter having a natural butter which is solid at 20°C and has a melting point below 50°C; and
e. about 50 to about 95 wt.% of water by weight of the total composition,
wherein the composition is in the form of an emulsion. Such composition is suitable for use on the skin. Surprisingly this combination of C₂₀₋₂₄ fatty alcohols, natural butter and succinoglycan was found to have no whitening or soapy effect on the skin. This combination provided a stable emulsion with a viscosity appropriate for a skin care moisturizer. Finally, this combination can be made of sustainably sourced ingredients.

As used herein, the term "emulsion" means a mixture of two phases, including a first phase and second phase, where the first phase is dispersed into the second phase. One of the phases is in the form of a liquid aqueous material, such as water. The second liquid phase may be a liquid, a solid, or a flowable solid, and includes one or more skin care components described herein, including, for example, emulsifying agents, thickeners, and humectants. The emulsion may be an oil-in-water emulsion.

Lotions and creams can be formulated as emulsions. Typically such lotions contain from 0.5% to about 5% of an emulsifier(s), while such creams would typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

The compositions described herein contain water as a cosmetically-acceptable carrier. As used herein, the term "cosmetically-acceptable carrier" means a carrier that is suitable for use in contact with the skin without undue toxicity, incompatibility, instability, irritation, allergic response, and the like. Water may be present in an amount ranging from about 50, 55, 60, 65, 70, 75, 80, 85, or 90 % to about 60, 65, 70, 75, 80, 85, 90 or 95% by weight of the total composition. In some embodiments, water is present in an amount ranging from about 60 to about 90, about 65 to about 85, about 70 to about 80 % by weight of the total composition.

### C₂₀₋₂₄ fatty alcohols;

The composition comprises one or more C₂₀₋₂₄ fatty alcohols. The one or more C₂₀₋₂₄ fatty alcohols may be selected from the group consisting of arachinyl alcohol (C20), heneicosyl alcohol (C21), behenyl alcohol (C22), lignoceryl alcohol (C24), and combinations thereof. The one or more C₂₀₋₂₄ fatty alcohols may be present in an amount of from about 1 to about 5 wt.%, or 1% to 4%, or even from 2% to 3% of by weight of the total composition.

In one or more embodiments, the composition is substantially free of C16 fatty alcohols, C18 fatty alcohols, or mixtures of C16/C18 alcohols. For example, cetearyl alcohol which is a mixture of cetyl (C16) alcohol and stearyl (C18) alcohol is preferably not present in the composition according to the invention.

### Anionic Surfactants

What is meant by a surfactant is a surface-active agent intended to cleanse or emulsify. Examples of suitable surfactants include those found in Chapter 37, pages 431-450 (Classification of surfactants, by L. Oldenhove de Guertechin) in Handbook of Cosmetic Science and Technology (edited by A. Barel, M. Paye and H. Maibach, Published in 2001 by Marcel Dekker, Inc., New York, NY) and include, but are not limited to anionic, amphoteric, nonionic, and cationic surfactants. As used herein, the term "anionic surfactant" refers to a surfactant having a negatively charged hydrophilic polar group. Anionic surfactants include citrates, phosphates, and sulfates, preferably citrates and phosphates. Examples of anionic surfactants include potassium cetyl phosphate, glyceryl stearate citrate, fatty acids in the anionic form, ammonium lauryl sulfate, sodium laureth sulfate, sodium lauryl sarcosinate, sodium myreth sulfate, sodium pareth sulfate, sodium stearate, sodium lauryl sulfate, α olefin sulfonate, and ammonium laureth sulfate. In further embodiments, the anionic surfactant is selected from potassium cetyl phosphate, glyceryl stearate citrate, and combinations thereof.

The anionic surfactant may be present in an amount ranging from about 0.01, 0.05, 0.1, 0.15, to about 1, 1.5, 2, 2.5 or 3 wt.%. In some embodiments, the anionic surfactant may be present in an amount ranging from about 0.1 to about 3 wt. % or about 0.2 to about 2 wt.%, or about 0.2 to about 1.5 wt.%.

### Thickening Polysaccharides

The compositions described herein comprise one or more thickening polysaccharides, wherein at least one of the thickening polysaccharides is succinoglycan. These thickening polysaccharides act as rheology modifiers. Examples of suitable polysaccharide thickeners aside from succinoglycan include various thickeners having molecular weights of greater than about 100,000 grams per mole, including chemistries such as: hydroxyalkyl cellulose; alkyl cellulose; hydroxyalkyl alkyl cellulose; xanthan and guar gums, carrageenan, alignates, pectin and mixtures thereof.

The one or more thickening polysaccharides comprising succinoglycan may be present in a total amount ranging from about 0.1 to about 0.5 wt.%, or about 0.2 to about 0.5 wt.%. The amount of succinoglycan in particular may range from about 0.05 to about 0.5 wt.%, or about 0.075 to about 0.4 wt.%, or about 0.1. to about 0.3 wt.%, or about 0.2 wt.%. Other preferred thickening polysaccharides include xanthan gum. When present, the xanthan gum may range from about 0.05 to about 0.5 wt.%, or about 0.075 to about 0.4 wt.%, or about 0.1. to about 0.3 wt.%, or about 0.2 wt.%. In a preferred embodiment, the composition comprises 0.2% succinoglycan + 0.2% xanthan gum, by weight % of the total composition.

### Emollients

The compositions described herein comprise one or more emollients comprising at least one natural butter having a natural butter which is solid at 20°C and has a melting point below 50°C. What is meant by an "emollient" is a compound that helps to maintain the soft, smooth, and pliable appearance of the skin (e.g., by remaining on the skin surface or in the stratum corneum to act as a lubricant). Examples of suitable emollients include those found in Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY).

The at least one natural butter having a natural butter which is solid at 20°C and has a melting point below 50°C may comprise shea butter, cacao butter, and combinations thereof. Other emollients include, but are not limited to, hexyldecyl stearate and plant, nut, and vegetable oils such as macadamia nut oil, rice bran oil, grape seed oil, palm oil, prim rose oil, hydrogenates peanut oil, and avocado oil. Other examples of suitable emollients include Candelilla Cera; Hydrogenated Vegetable oil; Olus Oil is sold under tradename Cegesoft VP by BASF.

The at least one natural butter may be present in amounts ranging from about 1.5 or 2 to about 3, 3.5, or 5 wt. %. The at least one natural butter may be present in amounts ranging from about 1.5 to about 5 wt.%, or about 2 to about 5 wt.%, or about 2 to about 3 wt.%.

The composition preferably contains more of the one or more C₂₀₋₂₄ fatty alcohols than the at least one natural butter. They may be present in a ratio of about 0.5 to about 2, or about 0.5 to about 1.8, or about 0.6 to about 1.5, or more preferably 1.5 of C₂₀₋₂₄ fatty alcohols to the at least one natural butter. In some embodiments, the C₂₀₋₂₄ fatty alcohols comprise behenyl alcohol and the emollient comprises shea butter. They may be present in a ratio of about 0.5 to about 2, or about 0.5 to about 1.8, or about 0.6 to about 1.5, or more preferably 1.5 of behenyl alcohol to shea butter. In one or more embodiments, the shea butter and behenyl alcohol are present in a total amount of about 3 to about 7 wt.%, or about 4 to about 6 wt.%, or about 5 wt.%.

The total amount of emollients may range from about 5, 6, 7, 8, 9, or 10 to about 13, 15, 18, or 20 wt. %. The total amount of emollients may range from about 5 to about 20 wt.%, or about 10 to about 20 wt.%, or about 10 to about 15 wt.%.

### Other Ingredients

The compositions of the present invention may comprise any of a variety of additional other auxiliary ingredients used conventionally in healthcare/personal care compositions (referred to generally as "personal care components"). These other personal care components nonexclusively include one or more, pearlescent or opacifying agents, conditioners, humectants, chelating agents, actives, exfoliants, and additives which enhance the appearance, feel and fragrance of the compositions, such as colorants, fragrances, preservatives, pH adjusting agents, rheology modifiers, and the like. Such auxiliary ingredients, when present, will be cosmetically / dermatologically acceptable and present in safe and effective amounts.

In addition to the anionic surfactants discussed above, the compositions described herein may contain additional surfactants, including other anionic, cationic, nonionic and amphoteric surfactants. Examples of suitable nonionic surfactants include, but are not limited to the fatty alcohol acid or amide ethoxylates, monoglyceride ethoxylates, sorbitan ester ethoxylates alkyl polyglycosides, and mixtures thereof. One suitable nonionic surfactant is the polyoxyethylene derivatives of polyol esters, wherein the polyoxyethylene derivative of polyol ester (1) is derived from (a) a fatty acid containing from about 8 to about 22, and preferably from about 10 to about 14 carbon atoms, and (b) a polyol selected from sorbitol, sorbitan, glucose, α-methyl glucoside, polyglucose having an average of about 1 to about 3 glucose residues per molecule, glycerin, pentaerythritol and mixtures thereof, (2) contains an average of from about 10 to about 120, and preferably about 20 to about 80 oxyethylene units; and (3) has an average of about 1 to about 3 fatty acid residues per mole of polyoxyethylene derivative of polyol ester.

Examples of polyoxyethylene derivatives of polyol esters include, but are not limited to PEG-80, sorbitan laurate, and polysorbate 20. PEG-80 sorbitan laurate, which is a sorbitan monoester of lauric acid ethoxylated with an average of about 80 moles of ethylene oxide, is available commercially from ICI Surfactants of Wilmington, Del. under the tradename, "Atlas G-4280." Polysorbate 20, which is the laurate monoester of a mixture of sorbitol and sorbitol anhydrides condensed with approximately 20 moles of ethylene oxide, is available commercially from ICI Surfactants of Wilmington, Del. under the tradename "Tween 20." Another class of suitable nonionic surfactants includes long chain alkyl glucosides or polyglucosides, which are the condensation products of (a) a long chain alcohol containing from about 6 to about 22, and preferably from about 8 to about 14 carbon atoms, with (b) glucose or a glucose-containing polymer. The alkyl gluocosides have about 1 to about 6 glucose residues per molecule of alkyl glucoside. A preferred glucoside is decyl glucoside, which is the condensation product of decyl alcohol with a glucose polymer and is available commercially from Henkel Corporation of Hoboken, N.J. under the tradename, "Plantaren 2000."

In some embodiments, the nonionic surfactant is selected from the group consisting of alkyl glucosides (e.g., decyl glucoside, lauryl glucoside, coco-glucoside) PEG-80, sorbitan Laurate and combinations thereof.

The compositions of the present invention may also contain an amphoteric surfactant. As used herein, the term "amphoteric" shall mean: 1) molecules that contain both acidic and basic sites such as, for example, an amino acid containing both amino (basic) and acid (e.g., carboxylic acid, acidic) functional groups; or 2) zwitterionic molecules which possess both positive and negative charges within the same molecule. The charges of the latter may be either dependent on or independent of the pH of the composition. The amphoteric surfactants are disclosed herein without a counter ion. One skilled in the art would readily recognize that under the pH conditions of the compositions of the present invention, the amphoteric surfactants are either electrically neutral by virtue of having balanced positive and negative charges, or they have counter ions such as alkali metal, alkaline earth, or ammonium counter ions. Examples of amphoteric surfactants suitable for use in the present invention include, but are not limited to, amphocarboxylates such as alkylamphoacetates (mono or di); alkyl betaines; alkylamidoalkyl betaines; alkylamidoalkyl sultaines; alkylamphophosphates; phosphorylated imidazolines such as phosphobetaines and pyrophosphobetaines; carboxyalkyl alkyl polyamines; alkylimino-dipropionates; alkylamphoglycinates (mono or di); alkylamphoproprionates (mono or di),); N-alkyl β-aminoproprionic acids; alkylpolyamino carboxylates; and mixtures thereof.

Classes of cationic surfactants that are suitable for use in this invention include alkyl quaternaries (mono, di, or tri), benzyl quaternaries, ester quaternaries, ethoxylated quaternaries, alkyl amines, and mixtures thereof, wherein the alkyl group has from about 6 carbon atoms to about 30 carbon atoms, with about 8 to about 22 carbon atoms being preferred. These cationic surfactants can be employed in composition of the present invention in an amount, based upon the total weight of the composition, from about 0.01% to about 18%, or from about 0.05% to about 15% or from about 0.1% to about 10%.

Any of a variety of commercially available pearlescent or opacifying agents which are capable of suspending water insoluble additives such as silicones and/or which tend to indicate to consumers that the resultant product is a conditioning shampoo are suitable for use in this invention. If used, the pearlescent or opacifying agent may be present in an amount, based upon the total weight of the composition, of from about 0.1 percent to about 10 percent, e.g. from about 1.5 percent to about 7 percent or from about 2 percent to about 5 percent. Examples of suitable pearlescent or opacifying agents include, but are not limited to mono or diesters of (a) fatty acids having from about 16 to about 22 carbon atoms and (b) either ethylene or propylene glycol; mono or diesters of (a) fatty acids having from about 16 to about 22 carbon atoms (b) a polyalkylene glycol of the formula: HO-(JO)a-H, wherein J is an alkylene group having from about 2 to about 3 carbon atoms; and a is 2 or 3; fatty alcohols containing from about 16 to about 22 carbon atoms; fatty esters of the formula: KCOOCH2L, wherein K and L independently contain from about 15 to about 21 carbon atoms; inorganic solids insoluble in the composition, and mixtures thereof.

Any of a variety of commercially available conditioners, such as volatile silicones, which impart additional attributes, such as gloss to the hair are suitable for use in this invention. The volatile silicone conditioning agent has an atmospheric pressure boiling point less than about 220 C. The volatile silicone conditioner may be present in the invention, in ranges of from about 0 percent (if unused) to about 3 percent, e.g. from about 0.25 percent to about 2.5 percent or from about 0.5 percent to about 1.0 percent, based on the overall weight of the composition. Examples of suitable volatile silicones nonexclusively include polydimethylsiloxane, polydimethylcyclosiloxane, hexamethyldisiloxane, cyclomethicone fluids such as polydimethylcyclosiloxane available commercially from Dow Corning Corporation of Midland, Mich. under the tradename, "DC-345" and mixtures thereof, such as cyclomethicone fluids. Other suitable secondary conditioners include cationic polymers, including polyquarterniums, cationic guar, and the like. The present inventive compositions may be free of added silicones.

Any of a variety of commercially available humectants, which are capable of providing moisturization and conditioning properties to the composition, are suitable for use in the present invention. What is meant by a humectant is a compound intended to increase the water content of the top layers of skin (e.g., hygroscopic compounds). Examples of suitable humectants include those found in Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY) and include, but are not limited to, glycerin, sorbitol or trehalose (e.g., α,α- trehalose, β,β-trehalose, α,β-trehalose) or a salt or ester thereof (e.g., trehalose 6-phosphate).

When present, the humectant may be present in an amount of from 1% to 10 %, preferably 4% to 6%, preferably about 5%, by weight % of the total composition.

Examples of suitable humectants nonexclusively include: 1) water soluble liquid polyols selected from the group comprising glycerine, propylene glycol, hexylene glycol, butylene glycol, dipropylene glycol, polyglycerols, and mixtures thereof; 2) polyalkylene glycol of the formula: HO-(R"O)b-H, wherein R" is an alkylene group having from about 2 to about 3 carbon atoms and b is an integer of from about 2 to about 10; 3) polyethylene glycol ether of methyl glucose of formula CH3-C6H10O5-(OCH2CH2)c-OH, wherein c is an integer from about 5 to about 25; 4) urea; and 5) mixtures thereof. The humectant is preferably chosen from glycerin.

Examples of suitable chelating agents include those which are capable of protecting and preserving the compositions of this invention. The chelating agent may include ethylenediamine tetracetic acid ("EDTA"), such as tetrasodium EDTA, available commercially from Dow Chemical Company of Midland, Mich. under the tradename, "Versene 100XL" and is present in an amount, based upon the total weight of the composition, from about 0 (if unused) to about 0.5 percent or from about 0.05 percent to about 0.25 percent. Suitable preservatives include, for example, parabens, quaternary ammonium species, phenoxyethanol, benzoates, sorbates, DMDM hydantoin, and are present in the composition in an amount, based upon the total weight of the composition, from about 0 (if unused) to about 1 percent or from about 0.05 percent to about 0.5 percent. The preservatives are preferably selected from the group consisting of potassium benzoate, potassium sorbate and combinations thereof. The preservatives may also be combined with other ingredients which provide a booster effect. One example of such a booster is caprylyl glyceryl ether, which is an emollient which can provide a boosting effect to potassium benzoate and/or potassium sorbate. The ratio of preservative to caprylyl glyceryl ether may be from about 0.3 to 0.9, preferably 0.6.

The composition may further optionally contain one or more benefit agents or pharmaceutically-acceptable salts thereof. As used herein, the term "benefit agent" includes any active ingredient that is to be delivered into and/or onto the skin, hair or nail at a desired location, such as a cosmetic agent or a pharmaceutical agent. By "cosmetic agent," it is meant any ingredient that is appropriate for cosmetically treating, providing nutrients to, and/or conditioning the hair, nail, and/or skin via topical application. By "pharmaceutical agent," it is mean any drug that is either hydrophobic or hydrophilic in nature and appropriate for topical use. As used herein "medicament agents" include those agents capable of promoting recovery from injury and illness. The benefit agent(s), if used, may be included in any of the first oil phase, the second oil phase, or the water phase, if compatible with the other components in the selected phase.

The benefit agents useful herein may be categorized by their therapeutic benefit or their postulated mode of action. However, it is to be understood that the benefit agents useful herein may, in some circumstances, provide more than one therapeutic benefit or operate via greater than one mode of action. Therefore, the particular classifications provided herein are made for the sake of convenience and are not intended to limit the benefit agents to the particular application(s) listed. In addition, the compounds, which are identified below as being suitable for use as benefit agents, may be used in an amount over and above the amount that they may be used for other purposes in the composition or personal care system.

Examples of suitable benefit agents include, but are not limited to, depigmentation agents; reflectants; film forming polymers; humectants; amino acids and their derivatives; antimicrobial agents; allergy inhibitors; anti-acne agents; anti-aging agents; anti-wrinkling agents, antiseptics; analgesics; antitussives; antipruritics; local anesthetics; anti-hair loss agents; hair growth promoting agents; hair growth inhibitor agents, antihistamines such as Mandragora Vernalis, Tanacetum Parthenium and the like; antiinfectives such as Acacia Catechu, Aloe Barbadensis, Convallaria Majalis, Echinacea, Eucalyptus, Mentha Piperita, Rosa Canina, Sassafras Albidum, and the like; inflammation inhibitors; anti-emetics; anticholinergics; vasoconstrictors; vasodilators; wound healing promoters; peptides, polypeptides and proteins; deodorants and anti-perspirants; medicament agents; skin emollients and skin moisturizers; skin firming agents, vitamins; tanning agents; skin lightening agents; antifungals such as Centaurea Cyanus, Kalmia Latifolia and antifungals for foot preparations; depilating agents; shaving preparations; external analgesics; perfumes; counterirritants; hemorrhoidals; insecticides; poison ivy products; poison oak products; burn products; anti-diaper rash agents; prickly heat agents; vitamins; amino acids and their derivatives; herbal extracts; retinoids; flavenoids; sensates; anti-oxidants; skin conditioners; hair lighteners, chelating agents; cell turnover enhancers; coloring agents; sunscreens, those active ingredients disclosed in U.S. Pat. No. 6,063,397, which is incorporated herein by reference, anti-edema agents, collagen enhancers, and mixtures thereof.

Examples of suitable anti-edema agents nonexclusively include bisabolol natural, synthetic bisabolol, and mixtures thereof.

Examples of suitable vasoconstrictors nonexclusively include horse chestnut extract, prickly ash, and mixtures thereof.

Examples of suitable anti-inflammatory agents nonexclusively include benoxaprofen, centella asiatica, bisabolol, feverfew (whole), feverfew (parthenolide free), green tea extract, green tea concentrate, hydrogen peroxide, lycopene including "Lyc-o-Pen" available from LycoRed Natural Products Industries, Ltd., oat oil, chamomile, and mixtures thereof. Examples of collagen enhancers nonexclusively include vitamin A, vitamin C, and mixtures thereof.

Examples of suitable skin firming agent nonexclusively include dimethylaminoethanol ("DMAE").

Examples of suitable antipruritics and skin protectants nonexclusively include oatmeal, betaglucan, feverfew, soy and derivatives thereof, bicarbonate of soda, colloidal oatmeal, surfactant based colloidal oatmeal cleanser, Anagallis Arvensis, Oenothera Biennis, Verbena Officinalis, and the like. These antipruritics may be used in an amount, based upon the total weight of the composition, from about 0.01 percent to about 40 percent, and preferably from about 1 percent to about 5 percent.

As used herein, colloidal oatmeal means the powder resulting from the grinding and further processing of whole oat grain meeting United States Standards for Number 1 or Number 2 oats. The colloidal oatmeal has a particle size distribution as follows: not more than 3 percent of the total particles exceed 150 micrometers in size and not more than 20 percent of the total particles exceed 75 micrometers in size. Examples of suitable colloidal oatmeals include, but are not limited to, "Tech-O" available from the Beacon Corporation and colloidal oatmeals available from Quaker.

Examples of suitable reflectants nonexclusively include mica, alumina, calcium silicate, glycol dioleate, glycol distearate, silica, sodium magnesium fluorosilicate, and mixtures thereof. Suitable film forming polymers include acetyl tyrosinamide, zinc pyrithione, coal tar, benzoyl peroxide, selenium sulfide, hydrocortisone, sulfur, menthol, pramoxine hydrochloride, tricetylmonium chloride, polyquaternium 10, panthenol, panthenol triacetate, vitamin A and derivatives thereof, vitamin B and derivatives thereof, vitamin C and derivatives thereof, vitamin D and derivatives thereof, vitamin E and derivatives thereof, vitamin K and derivatives thereof, keratin, lysine, arginine, hydrolyzed wheat proteins, hydrolyzed silk proteins, octyl methoxycinnamate, oxybenzone, minoxidil, titanium dioxide, zinc dioxide, retinol, erthromycin, tretinoin, and mixtures thereof.

One type of benefit agent includes those therapeutic components that are effective in the treatment of dandruff, seborrheic dermatitis, and psoriasis as well as the symptoms associated therewith. Examples of such suitable benefits agents nonexclusively include zinc pyrithione, anthralin, shale oil and derivatives thereof such as sulfonated shale oil, selenium sulfide, sulfur; salicylic acid; coal tar; povidone-iodine, imidazoles such as ketoconazole, dichlorophenyl imidazolodioxalan, which is commercially available from Janssen Pharmaceutica, N.V., under the tradename, "Elubiol", clotrimazole, itraconazole, miconazole, climbazole, tioconazole, sulconazole, butoconazole, fluconazole, miconazole nitrate and any possible stereo isomers and derivatives thereof; piroctone olamine (Octopirox); selenium sulfide; ciclopirox olamine; anti-psoriasis agents such as vitamin D analogs, e.g. calcipotriol, calcitriol, and tacaleitrol; vitamin A analogs such as esters of vitamin A, e.g. vitamin A palmitate, retinoids, retinols, and retinoic acid; corticosteroids such as hydrocortisone, clobetasone, butyrate, clobetasol propionate and mixtures thereof.

### Dermatological Product Forms

The composition may be applied topically. Such topical application may be to any skin in need of treatment on the body, for example skin of the face, lips, neck, chest, back, buttocks, arms, axilla, and/or legs. The composition may also be administered to a mucous membrane (i.e., in the oral cavity).

In some embodiments, the present invention is in the form of a substrate comprising a composition of the present invention. Any suitable substrate may be used. Examples of suitable substrates and substrate materials are disclosed, for example, in US7452547 and US2009/0241242 which are incorporated herein by reference in their entirety. In some embodiments, the composition is in the form of a tablet, pill, or capsule. In one or more embodiments, the composition is in the form of a solution, suspension, emulsion, lotion, cream, serum, gel, stick, spray, ointment, liquid wash, soap bar, shampoo, hair conditioner, paste, foam, powder, mousse, shaving cream, hydrogel, or film-forming product.

Any suitable method of applying the composition to the skin in need may be used. For example, the composition may be applied directly from a package to the skin in need, by hand to the skin in need, or may be transferred from a substrate such as a wipe or mask, or a combination of two or more thereof. In other embodiments, the composition may be applied via a dropper, tube, roller, spray, and patch or added to a bath or otherwise to water to be applied to the skin, and the like. The composition may be applied in a variety of manners or forms, including, without limitation, as a leave-on cream, mask, and /or serum.

While the foregoing description represent exemplary embodiments of the present invention, it will be understood that various additions, modifications and substitutions may be made therein without departing from the spirit and scope of the present invention. In particular, it will be clear to those skilled in the art that the present invention may be embodied in other specific forms, structures, arrangements, proportions, and with other elements, materials, and components, without departing from the spirit or essential characteristics thereof. One skilled in the art will appreciate that the invention may be used with many modifications of structure, arrangement, proportions, materials, and components and otherwise, used in the practice of the invention, which are particularly adapted to specific environments and operative requirements without departing from the principles of the present invention. The presently disclosed embodiments are therefore to be considered in all respects as illustrative and not restrictive, the scope of the invention being indicated by the appended claims, and not limited to the foregoing description. It will be appreciated that in the claims, the term "comprises/comprising" does not exclude the presence of other elements or steps. In addition, singular references do not exclude a plurality. The terms "a", "an", "first", "second", etc., do not preclude a plurality.

All percentages, parts and ratios are based upon the total weight of the composition of the present invention, unless otherwise specified. All such weights as they pertain to the listed ingredients are based on the level of the particular ingredient described and, therefore, do not include carriers or by-products that may be included in commercially available materials, unless otherwise specified.

### Exemplary Embodiments

The above embodiments may be combined in any suitable fashion. Thus, for example, in one aspect, the invention pertains to a composition comprising:
a. about 1 to about 5 wt.% of one or more C20-24 fatty alcohols by weight of the total composition;
b. about 0.2 to about 3 wt.% of one or more anionic surfactants by weight of the total composition;
c. about 0.05 to about 2 wt.% of one or more thickening polysaccharides comprising succinoglycan by weight of the total composition;
d. about 1 to about 20 wt. % of one or more emollients comprising at least at least one natural butter having a natural butter which is solid at 20°C and has a melting point below 50°C by weight of the total composition; and
e. about 50 to about 95 wt.% of water by weight of the total composition,
wherein the composition is in the form of an emulsion.

In further embodiments, the composition comprises:
a. about 1 to about 5 wt.% of behenyl alcohol by weight of the total composition;
b. about 0.2 to about 3 wt.% of potassium cetyl phosphate by weight of the total composition;
c. about 0.05 to about 1 wt.% of succinoglycan by weight of the total composition;
d. about 0.05 to about 1 wt.% of xanthan gum by weight of the total composition
e. about 1 to about 20 wt. % of one or more emollients comprising shea butter; and
f. about 70 to about 80 wt.% of water by weight of the total composition.

As used herein, "essentially free" or "substantially free" of an ingredient means containing less than 0.1 weight percent, or less than 0.01 weight percent, or none of an ingredient.

To provide a more concise description, some of the quantitative expressions given herein are not qualified with the term "about". It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

To provide a more concise description, some of the quantitative expressions herein are recited as a range from about amount X to about amount Y. It is understood that wherein a range is recited, the range is not limited to the recited upper and lower bounds, but rather includes the full range from about amount X through about amount Y, or any amount or range therein.

### Experimental Part

### Materials

- Water is demineralized water,
- Glycerin was purchased from Oleon, Avril Group France,
- Aloe Barbadensis Leaf Juice is sold under tradename Terra-Pure^{™} Spray Dried Aloe Vera Juice Powder 200X (TN001) by Terry Laboratories, Melbourne (FL) USA,
- Potassium Cetyl Phosphate, Hydrogenated Palm Glycerides is sold under tradename Emulsiphos 677660 from Symrise, active matter content is 100%,
- Glyceryl Stearate Citrate is sold under tradename DERMOFEEL GSC by Evonik, active matter content is 100%,
- Lauryl Glucoside and Polyglyceryl-2 Dipolyhydroxystearate is sold under tradename Eumulgin VL 75 by BASF. Active matter content is 60%, the remaining are 20% water and 20% glycerin,
- Cetearyl Alcohol is sold under tradename Lanette O by BASF, active matter content is 100%,
- Behenyl Alcohol is sold under tradename Lanette 22 by BASF, active matter content is 100%,
- Xanthan Gum is sold under tradename Keltrol CG-LAX-T by CP Kelco, active matter content is 100%,
- Succinoglycan is sold under tradename Rheozan SH by Solvay, active matter content is 100%,
- Glyceryl stearate is sold under tradename Cutina GMS V by BASF, active matter content is 100%,
- Candelilla Cera; Hydrogenated Vegetable oil; Olus Oil is sold under tradename Cegesoft VP by BASF, active matter content is 100%,
- Cocoglycerides is sold under tradename Myritol 331 by BASF, active matter content is 100%,
- Butyrospermum Parkii (Shea) Butter is sold under tradename Cetiol SB 45 by BASF, active matter content is 100%,
- Sodium benzoate is sold under tradename Ronacare Sodium benzoate by Merck, active matter content is 100%,
- Potassium sorbate was purchased from Merck, active matter content is 100%,
- Caprylyl Glyceryl Ether is sold under tradename Velsan CGE by Clariant International, active matter content is 100%.
- Citric acid was purchased from Cellmark.

### Methods

Standard formulation protocol for the inventive examples 6 to 8:
- 22% (% relative to the total formulation weight) of demineralized water and Aloe Barbadensis Leaf Juice are mixed at room temperature, then add
- additional 25% (% relative to the total formulation weight) of demineralized water and glycerin, then Succinoglycan and Xanthan Gum,
- Start heating to 80°C, while heating add an additional 10% of demineralized water,
- Between 70°C and 80°C, add Cocoglycerides, optionally add Lauryl Glucoside and Polyglyceryl-2 Dipolyhydroxystearate,
- When 80°C is reached add Potassium Cetyl Phosphate and Hydrogenated Palm Glycerides; Behenyl Alcohol; Candelilla Cera, Hydrogenated Vegetable oil and Olus Oil; Butyrospermum Parkii (Shea) Butter and optionally Glyceryl Stearate Citrate,
- Mix at 80°C for 15 minutes then start to cool down to 25°C,
- While cooling down, when the temperature reaches 60°C, add Potassium Sorbate and Caprylyl Glyceryl Ether; optionally Sodium benzoate; then the remaining amount of demineralized water.
- When 25°C is reached, adjust the pH to 4.7 with a 50% citric acid solution.

All formulations were evaluated for whitening effect, stability and viscosity. "Whitening effect" refers to the propensity of a formulation to leave a white film on the skin after application. Whitening effect was evaluated by visual observation. Stability of the formulations were done through visual evaluation after 3 months at 40 °C.

Viscosities were measured with a rheometer (Physica) at 20°C, with a shear rate at 45s⁻¹. Viscosity is an important feature to skin care formulas, as they must have the right consistency to be pleasing to the consumer, while also being able to be dispensed correctly (e.g., being able to be pumped). Generally speaking, a viscosity range of about 400 to about 5000 mPa.s⁻¹ is considered acceptable for a skin care composition, or an upper limit of 3,000 mPa.s⁻¹ for a pumpable skin care composition.

### Comparative examples

| INCI Name (active matter %) | Ex. 1 | Ex. 2 | Ex. 3 | Ex. 4 | Ex. 5 |
|---|---|---|---|---|---|
| Water | 77,99 | 80,64 | 77,64 | 76,64 | 76,64 |
| Glycerin | 5 | 5 | 5 | 5 | 5 |
| Aloe Barbadensis Leaf Juice | 0,11 | 0,11 | 0,11 | 0,11 | 0,11 |
| Potassium Cetyl Phosphate, Hydrogenated Palm Glycerides | 1 | 1 | 1 | 1 | 1 |
| Cetearyl Alcohol | 2 | 0 | 0 | 2 | 1 |
| Behenyl Alcohol | 0 | 1 | 3 | 1 | 2 |
| Xanthan Gum | 0,2 | 0,2 | 0,2 | 0,4 | 0,4 |
| Succinoglycan | 0,2 | 0,2 | 0,2 | 0 | 0 |
| Glyceryl stearate | 1,5 | 0 | 0 | 0 | 0 |
| Candelilla Cera; Hydrogenated Vegetable oil; Olus Oil | 6 | 6 | 6 | 6 | 6 |
| Cocoglycerides | 5 | 5 | 5 | 5 | 5 |
| Butyrospermu m Parkii (Shea) Butter | 0 | 0 | 1 | 2 | 2 |
| Potassium sorbate | 0,15 | 0,15 | 0,15 | 0,15 | 0,15 |
| Caprylyl Glyceryl Ether | 0,5 | 0,5 | 0,5 | 0,5 | 0,5 |
| Citric Acid | 0,35 | 0,2 | 0,2 | 0,2 | 0,2 |
| Visco T0 (mPa.s⁻¹) | 684 | 442 | 868 | 725 | 647 |
| Whitening effect | White film formation observed | Low white film observed | None | White film observed | White film observed |
| Stability (Acceptable?) | Yes | Yes | Formation of solid particles | Yes | Yes |

- Example 1 : Considered to be comparative because it did not contain one or more a C₂₀₋₂₄ fatty alcohols or at least one natural butter having a natural butter which is solid at 20°C and has a melting point below 50°C.
- Example 2 : Considered to be comparative because it did not contain at least one natural butter having a natural butter which is solid at 20°C and has a melting point below 50°C.
- Example 3 : Considered to be comparative in some embodiments because the ratio of behenyl alcohol to shea butter is 3.
- Examples 4 & 5: Considered to be comparative because it did not contain one or more thickening polysaccharides comprising succinoglycan.

### Inventive examples

| INCI Name (% active matter) | Ex. 6 | Ex. 7 | Ex. 8 | Ex. 9 | Ex. 10 | Ex. 11 |
|---|---|---|---|---|---|---|
| Water | 76,64 | 76,64 | 76,34 | 75,61 | 75,89 | 75,9 9 |
| Glycerin | 5 | 5 | 5 | 5 | 5 | 5,1 |
| Aloe Barbadensis Leaf Juice | 0,11 | 0,11 | 0,11 | 0,11 | 0,11 | 0,11 |
| Potassium Cetyl Phosphate, Hydrogenated Palm Glycerides | 1 | 1 | 1 | 1 | 1 | 1 |
| Glyceryl Stearate Citrate | 0 | 0 | 0 | 0 | 0,5 | 0 |

| Lauryl Glucoside, Polyglyceryl-2 Dipolyhydroxystearate | 0 | 0 | 0 | 0 | 0 | 0,3 |
|---|---|---|---|---|---|---|
| Behenyl Alcohol | 2 | 3 | 3 | 3 | 3 | 3 |
| Xanthan Gum | 0,2 | 0,2 | 0,2 | 0,2 | 0,2 | 0,2 |
| Succinoglycan | 0,2 | 0,2 | 0,2 | 0,2 | 0,2 | 0,2 |
| Candelilla Cera; Hydrogenated Vegetable oil; Olus Oil | 6 | 6 | 6 | 6 | 6 | 6 |
| Cocoglycerides | 5 | 5 | 5 | 5 | 5 | 5 |
| Butyrospermum Parkii (Shea) Butter | 3 | 2 | 2 | 2 | 2 | 2 |
| Sodium benzoate | 0 | 0 | 0,3 | 0 | 0 | 0 |
| Potassium sorbate | 0,15 | 0,15 | 0,15 | 0,3 | 0,3 | 0,3 |
| Caprylyl Glyceryl Ether | 0,5 | 0,5 | 0,5 | 0,5 | 0,5 | 0,5 |
| Fragrance | 0 | 0 | 0 | 0,78 | 0 | 0 |
| Citric Acid | 0,2 | 0,2 | 0,2 | 0,3 | 0,3 | 0,3 |
| | | | | | | |
| Ratio behenyl / shea butter | 0,67 | 1,5 | 1,5 | 1,5 | 1,5 | 1,5 |
| Ratio Preservative / booster | 0,3 | 0,3 | 0,9 | 0,6 | 0,6 | 0,6 |
| | | | | | | |
| Visco T0 (mPa.s⁻¹) | 818 | 924 | 578 | 487 | 403 | 623 |
| Whitening Effect | None | None | None | None | None | None |
| Stability (Acceptable?) | Yes | Yes | Yes | Yes | Yes | Yes |

As can be seen from the above examples, only the inventive examples demonstrated sufficient viscosity and stability without producing a whitening effect. This is surprising because it is difficult to find the right selection of ingredients which achieve the desired level of stability, texture and sensory characteristics, with no suggestions in the literature on how to achieve this balance.

The invention provides the following numbered aspects:
1. A composition comprising:
   a. one or more a C₂₀₋₂₄ fatty alcohols;
   b. one or more anionic surfactants;
   c. one or more thickening polysaccharides comprising succinoglycan;
   d. one or more emollients comprising at least one natural butter having a natural butter which is solid at 20°C and has a melting point below 50°C; and
   e. about 50 to about 95 wt.% of water by weight of the total composition,
   wherein the composition is in the form of an emulsion.
2. The composition of aspect 1, wherein the one or more C₂₀₋₂₄ fatty alcohols is selected from the group consisting of behenyl alcohol, arachinyl alcohol, heneicosanol, Lignoceryl alcohol and combinations thereof.
3. The composition of aspect 1 or 2, wherein the one or more C₂₀₋₂₄ fatty alcohols is present in an amount of from about 1 to about 5 wt.% of by weight of the total composition.
4. The composition of any of aspects 1-3, wherein the one or more anionic surfactants is selected from the group consisting of potassium cetyl phosphate, glyceryl stearate citrate, fatty acids in the anionic form, and combinations thereof.
5. The composition of any of aspects 1-4, wherein the one or more one or more anionic surfactants is present in an amount of from about 0.1 to about 3 wt.% of by weight of the total composition.
6. The composition of any of aspects 1-5, wherein the one or more thickening polysaccharides further comprises xanthan gum, hydroxyalkyl cellulose, alkyl cellulose, hydroxyalkyl alkyl cellulose, guar gum, carrageenan, alignates, pectin, and combinations thereof.
7. The composition of any of aspects 1-7, wherein the one or more thickening polysaccharide is present in an amount of from about 0.05 to about 2 wt.% of by weight of the total composition.
8. The composition of any of aspects 1-7, wherein at least one natural butter having a natural butter which is solid at 20°C and has a melting point below 50°C is present in an amount ranging from about 1.5 to about 5 wt.%.
9. The composition of any of aspects 1-8, wherein the at least one natural butter having a natural butter which is solid at 20°C and has a melting point below 50°C comprises shea butter.
10. The composition of any of aspects 1-9, wherein the emollient is selected from the group consisting of shea butter, cocoglyceride, candelilla cera and hydrogenated vegetable oil, olus oil; caprylyl glyceryl ether, and combinations thereof.
11. The composition of any of aspects 1-10, wherein the one or more C₂₀₋₂₄ fatty alcohols comprise behenyl alcohol, the one or more emollients comprises shea butter, and the behenyl alcohol and shea butter are present in a ratio of about 0.5 to about 2.
12. The composition of any of aspects 1-11, further comprising a non-ionic surfactant.
13. A composition comprising:
   a. about 1 to about 5 wt.% of one or more C₂₀₋₂₄ fatty alcohols by weight of the total composition;
   b. about 0.2 to about 3 wt.% of one or more anionic surfactants by weight of the total composition;
   c. about 0.05 to about 2 wt.% of one or more thickening polysaccharides comprising succinoglycan by weight of the total composition;
   d. about 1 to about 20 wt. % of one or more emollients comprising at least one natural butter having a natural butter which is solid at 20°C and has a melting point below 50°C by weight of the total composition; and
   e. about 50 to about 95 wt.% of water by weight of the total composition,
   wherein the composition is in the form of an emulsion.
14. The composition of aspect 13, wherein the composition comprises:
   a. about 1 to about 5 wt.% of behenyl alcohol by weight of the total composition;
   b. about 0.2 to about 3 wt.% of potassium cetyl phosphate by weight of the total composition;
   c. about 0.05 to about 1 wt.% of succinoglycan by weight of the total composition;
   d. about 0.05 to about 1 wt.% of xanthan gum by weight of the total composition
   e. about 1 to about 20 wt. % of one or more emollients comprising shea butter; and
   f. about 70 to about 80 wt.% of water by weight of the total composition.
15. A method of treating skin, the method comprising: applying the composition of any of aspects 1-14 to skin.

## Claims

1. A composition comprising:
a. one or more a C₂₀₋₂₄ fatty alcohols;
b. one or more anionic surfactants;
c. one or more thickening polysaccharides comprising succinoglycan;
d. one or more emollients comprising at least one natural butter having a natural butter which is solid at 20°C and has a melting point below 50°C; and
e. about 50 to about 95 wt.% of water by weight of the total composition,
wherein the composition is in the form of an emulsion.

2. The composition of claim 1, wherein the one or more C₂₀₋₂₄ fatty alcohols is selected from the group consisting of behenyl alcohol, arachinyl alcohol, heneicosanol, Lignoceryl alcohol and combinations thereof.

3. The composition of claim 1 or 2, wherein the one or more C₂₀₋₂₄ fatty alcohols is present in an amount of from about 1 to about 5 wt.% of by weight of the total composition.

4. The composition of any of claims 1-3, wherein the one or more anionic surfactants is selected from the group consisting of potassium cetyl phosphate, glyceryl stearate citrate, fatty acids in the anionic form, and combinations thereof.

5. The composition of any of claims 1-4, wherein the one or more one or more anionic surfactants is present in an amount of from about 0.1 to about 3 wt.% of by weight of the total composition.

6. The composition of any of claims 1-5, wherein the one or more thickening polysaccharides further comprises xanthan gum, hydroxyalkyl cellulose, alkyl cellulose, hydroxyalkyl alkyl cellulose, guar gum, carrageenan, alignates, pectin, and combinations thereof.

7. The composition of any of claims 1-7, wherein the one or more thickening polysaccharide is present in an amount of from about 0.05 to about 2 wt.% of by weight of the total composition.

8. The composition of any of claims 1-7, wherein at least one natural butter having a natural butter which is solid at 20°C and has a melting point below 50°C is present in an amount ranging from about 1.5 to about 5 wt.%.

9. The composition of any of claims 1-8, wherein the at least one natural butter having a natural butter which is solid at 20°C and has a melting point below 50°C comprises shea butter.

10. The composition of any of claims 1-9, wherein the emollient is selected from the group consisting of shea butter, cocoglyceride, candelilla cera and hydrogenated vegetable oil, olus oil; caprylyl glyceryl ether, and combinations thereof.

11. The composition of any of claims 1-10, wherein the one or more C₂₀₋₂₄ fatty alcohols comprise behenyl alcohol, the one or more emollients comprises shea butter, and the behenyl alcohol and shea butter are present in a ratio of about 0.5 to about 2.

12. The composition of any of claims 1-11, further comprising a non-ionic surfactant.

13. A composition comprising:
a. about 1 to about 5 wt.% of one or more C₂₀₋₂₄ fatty alcohols by weight of the total composition;
b. about 0.2 to about 3 wt.% of one or more anionic surfactants by weight of the total composition;
c. about 0.05 to about 2 wt.% of one or more thickening polysaccharides comprising succinoglycan by weight of the total composition;
d. about 1 to about 20 wt. % of one or more emollients comprising at least one natural butter having a natural butter which is solid at 20°C and has a melting point below 50°C by weight of the total composition; and
e. about 50 to about 95 wt.% of water by weight of the total composition,
wherein the composition is in the form of an emulsion.

14. The composition of claim 13, wherein the composition comprises:
a. about 1 to about 5 wt.% of behenyl alcohol by weight of the total composition;
b. about 0.2 to about 3 wt.% of potassium cetyl phosphate by weight of the total composition;
c. about 0.05 to about 1 wt.% of succinoglycan by weight of the total composition;
d. about 0.05 to about 1 wt.% of xanthan gum by weight of the total composition
e. about 1 to about 20 wt. % of one or more emollients comprising shea butter; and
f. about 70 to about 80 wt.% of water by weight of the total composition.

15. A method of treating skin, the method comprising: applying the composition of any of claims 1-14 to skin.
